# EUROPÄISCHE PATENTSCHRIFT

(11) **EP 1 232 741 B1**
(45) Veröffentlichungstag und Bekanntmachung des Hinweises auf die Patenterteilung: **26.03.2003**
(21) Anmeldenummer: 02002929.4
(22) Anmeldetag: 09.02.2002
(51) Int. Cl.: A61K 7/13

(54) **Ubichinon-Haarfärbemittel**
Ubiquinone hair dyes
Colorants capillaires ubiquinoniques

(30) Priorität: 14.02.2001 DE 10106746
(43) Veröffentlichungstag der Anmeldung: 21.08.2002
(73) Patentinhaber: KPSS Kao Professional Salon Services GmbH, 64280 Darmstadt (DE)
(72) Erfinder: Nöcker, Bernd, Dr., 64372 Ober-Ramstadt (DE); Garbe, Barbara, 64625 Bensheim (DE); Wilz, Rüdiger, 64297 Darmstadt (DE); Ghiasi, Fariba, 60314 Frankfurt (DE)

(56) Entgegenhaltungen:
- EP-A- 1 059 079
- EP-A- 1 059 080
- EP-A- 1 059 081
- DE-A- 4 335 624
- US-A- 4 713 397
- US-A- 5 064 442
- US-A- 6 045 826
- PATENT ABSTRACTS OF JAPAN vol. 010, no. 178 (C-355), 21. Juni 1986 (1986-06-21) & JP 61 027914 A (SHISEIDO CO LTD), 7. Februar 1986 (1986-02-07)

## Beschreibung

Die vorliegende Erfindung betrifft ein Haarfärbemittel auf Basis von Oxidationsfarbstoff-Vorprodukten mit verbesserten Gebrauchseigenschaften, insbesondere gesteigerten Färbeeigenschaften.
Oxidationsfarbstoff-Haarfärbemittel sind seit Jahrzehnten bekannt und bewährt, gleichwohl jedoch auch noch verbesserungsfähig, insbesondere hinsichtlich der Eigenschaften der durch sie erzielten Haarfärbungen, vor allem Farbintensität und Farbstabilität, d.h. Beständigkeit gegen Haarwäsche, Dauerwellbehandlung und Umwelteinflüsse wie UV-Strahlen und chemische Beeinflussungen, insbesondere bei geschädigtem Haar.
Gelöst wird diese Aufgabe durch den Zusatz von 0,001 bis 10 Gew.-%, berechnet auf die Gesamtzusammensetzung, mindestens eines Ubichinons der Formel 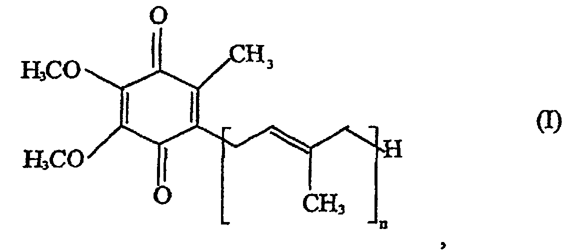 worin n eine Zahl von 1 bis 10 bedeutet.
Bevorzugte Ubichinone sind solche mit n = 6 bis 10, insbesondere Ubichinon 50, worin n 10 bedeutet, auch unter der Bezeichnung "Coenzym Q 10" bekannt.
Die Verwendung von "Coenzym Q 10" in kosmetischen Mitteln, insbesondere in Haarpflegemitteln, ist an sich bekannt.
Die EP 0 751 762 B beschreibt Haarpflegemittel, die ein oder mehrere Ubichinone, insbesondere Coenzym Q 6 und Coenzym Q 10, in Kombination mit Retinolen, Retinalen und β-Carotin, zur Behandlung der Hautalterung enthalten.
Aus der DE 199 26 167 A1 sind Stylingmittel auf Wasserbasis bekannt, die ein oder mehrere Biochinone wie Ubichinone und Plastochinone zum Schutz der Kopfhaut und des Haares vor unerwünschten Oxidationsprozessen enthalten.
Die DE 199 26 168 A1 offenbart Stylingmittel auf alkoholischer Basis mit Biochinonen, insbesondere Coenzym Q 10.

Die DE 199 26 170 A1 betrifft die Verwendung eines oder mehrerer Biochinone wie Ubichinone in kosmetischen Haarreinigungsmitteln.
Schließlich beschreibt die DE 199 26 156 A1 die Verwendung von Biochinonen wie Ubichinon zur Herstellung von kosmetischen Zubereitungen zur Verbesserung der Haarstruktur, insbesondere der Kämmbarkeit des Haares, also haarkonditionierenden Zusammensetzungen.

Gegenüber diesem Stand der Technik war die farbverbessemde Wirkung des Zusatzes von Ubichinonen in Oxidationshaarfärbemitteln, insbesondere bei Anwendung auf vorgeschädigtem Haar, wobei zusätzlich auch noch dessen kosmetische Eigenschaften wie Glanz, Griff und Sprungkraft verbessert werden, nicht vorhersehbar.

Die bevorzugte Einsatzkonzentration der Coenzyme Q1 bis Q 10 beträgt etwa 0,005 bis 5, insbesondere etwa 0,01 bis 2,5, besonders bevorzugt etwa 0,05 bis 1 Gew.-%, berechnet auf die das Oxidationsfarbstoff-Vorprodukt (ohne Oxidationsmittel) enthaltende Zusammensetzung.
Als solche können die üblichen Entwickler- und Kupplersubstanzen enthaltenden Produkte zum Einsatz gelangen, die dem Fachmann per se bekannt sind, beispielsweise aus der Monographie von K. Schrader, Grundlagen und Rezepturen der Kosmetika, 2. Aufl. (Hüthig Buchverlag, 1989), S. 782-804.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 4-Aminophenol, 4-Amino-3-methylphenol, 2-(2-Hydroxyethylamino)-5-aminotoluol und 1-Amino-4-bis- (2'-hydroxyethyl)-aminobenzol bzw. deren wasserlöslichen Salze; beispielhafte Kupplersubstanzen sind Resorcin, 2-Methylresorcin, 4-Chlorresorcin, 2-Amino-4-chlorphenol, 4-(N-methyl)-aminophenol, 2-Aminophenol, 3-Aminophenol, 1-Methyl-2-hydroxy-4-aminobenzol, 3-N,N-Dimethylaminophenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 1,4-Diamino-2-chlorbenzol, 4,6-Dichlorresorcin, 1,3-Diaminotoluol, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxynaphthalin, 2,4-Diamino-3-chlorphenol und/oder 1-Methoxy-2-amino-4-(2'-hydroxyethylamino)-benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbmischungen kann jeweils etwa 0,25 bis etwa 5 Gew.-%, je nach gewünschter Färbung betragen.

Als Oxidationsmittel, die mit der die das Oxidationsfarbstoffvorprodukt-System enthaltenden Zusammensetzung vor der Anwendung auf dem Haar vermischt werden, werden vor allem verdünnte Wasserstoffperoxid-Lösungen, -Emulsionen oder -Gele eingesetzt, möglich, aber weniger üblich ist auch die Verwendung weiterer Peroxide wie Erdalkaliperoxide, Harnstoffperoxid, Melaminperoxid, etc. in entsprechenden stöchiometrischen Mengen.
Die Oxidationsfarbstoffzusammensetzungen können als Lösungen, Cremes, Pasten, Gele, Aerosole, etc. Verwendung finden.

Der pH-Wert der gebrauchsfertigen Zusammensetzung kann im alkalischen, neutralen oder sauren Bereich liegen.

Die Einwirkungszeit auf dem Haar liegt vorzugsweise bei jeweils etwa fünf bis dreißig Minuten, insbesondere bei jeweils etwa 10 bis 20, beispielsweise jeweils 15 Minuten für die alkalische oder angesäuerte Farbmischung.
Das erfindungsgemäße Haarfärbemittel eignet sich dabei sowohl zum ganzheitlichen, d.h. erstmaligen Färben der Haare, als auch zum Nachfärben.

Die erfindungsgemäß eingesetzten Mittel können selbstverständlich alle in Haarfärbemitteln üblichen Stoffe enthalten, auf deren detaillierte Aufzählung hier verzichtet wird, und als (wäßrige) Lösungen, Emulsionen, Cremes, Schäume etc. vorliegen.
Auch die Mitverwendung direktziehender Haarfarbstoffe zur Nuancierung der erwünschten Farbtöne ist möglich.
Zur Vermeidung von Wiederholungen wird hierzu auf den Stand der Technik verwiesen, wie er beispielsweise in der Monographie von K. Schrader, l.c., beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Die folgenden Beispiele dienen der Illustration der Erfindung.

### Beispiel 1

Im Halbseitenversuch wurde auf eine Hälfte gewaschenen Haares eine alkalische Färbezusammensetzung, die durch Vermischen von gleichen Gewichtsteilen einer 6%-igen Wasserstoffperoxidlösung und eines Oxidationsfarbstoffvorprodukts der folgenden Zusammensetzung erhalten wurde, aufgebracht

| **Trägermasse** | |
|---|---|
| Cetylstearylalkohol | 11,00 (Gew.-%) |
| Oleth-5 | 5,00 |
| Ölsäure | 2,50 |
| Stearinsäuremonoethanolamid | 2,50 |
| Cocosfettsäuremonoethanolamid | 2,50 |
| Natriumlaurylsulfat | 1,70 |
| Natriumsulfit | 1,00 |
| 1,2-Propandiol | 1,00 |
| Ascorbinsäure | 0,50 |
| Ammoniumchlorid | 0,50 |
| EDTA, Tetranatriumsalz | 0,20 |
| Parfum | 0,40 |
| Weizenproteinhydrolysat | 0,20 |
| Silica | 0,10 |
| Coenzym Q 10 | 0,05 |

| **Oxidationsfarbstoffmischung** | |
|---|---|
| 2,5,6-Triamino-4-hydroxypyrimidinsulfat | 0,01 |
| 2,5-Diaminotoluolsulfat | 0,55 |
| 4-Chlorresorcin | 0,17 |
| Resorcin | 0,05 |
| 3-Aminophenol | 0,03 |
| Ammoniak | ad pH 9,8 |
| Wasser | ad 100,00 |

Nach zwanzigminütiger Einwirkung wurde gewaschen, gespült und getrocknet.

Es wurde eine glänzende, intensive, gleichmäßige Mittelblondfärbung mit einem weichen Griff des Haares erhalten.

Das Weglassen des Coenzyms in der zur Färbung der anderen Haarhälfte benutzten Zusammensetzung führte zu einer deutlich weniger intensiven und gleichmäßigen Färbung mit verringertem Glanz.
Darüberhinaus waren sowohl die Naß- als auch die Trockenkämmbarkeit sowie der Griff des Haares deutlich schlechter.

### Beispiel 2

In eine der Zusammensetzung nach Beispiel 1 entsprechende Trägermasse, die jedoch als Ubichinon 0,03 Gew.-% Coenzym Q 6 enthielt, wurde die folgende Oxidationsfarbstoffmischung eingebracht:

| | |
|---|---|
| 2,5,6-Triamino-4-hydroxypyrimidinsulfat | 0,01 (Gew.-%) |
| 2,5-Diaminotoluolsulfat | 0,90 |
| 2-Amino-4-hydroxypyridin | 0,80 |
| 1-Naphthol | 0,17 |
| 3-Aminophenol | 0,10 |
| Resorcin | 0,03 |

Dieses Produkt wurde mit 6%-iger H₂O₂-Lösung im Gewichtsverhältnis 1:1 vermischt (pH 9,5) und auf eine Haarhälfte aufgebracht.
Die andere Haarhälfte wurde mit einer Zusammensetzung ohne Coenzym Q6 behandelt.
Nach 20-minütiger Einwirkung wurde mit Wasser ausgewaschen, shampooniert und getrocknet.
Es wurde eine intensive, glänzende, gleichmäßige Mahagoni-Färbung erhalten.
Die mit der Zusammensetzung ohne Coenzym Q 6 behandelte Haarhälfte wies nicht nur eine weniger intensive und gleichmäßige Färbung, sondern auch einen rauhen Griff auf.

### Beispiel 3

Die in Beispiel 1 beschriebene Zusammensetzung wurde dahingehend variiert, daß sie so eingestellt war, daß beim Vermischen mit 6%-iger H₂O₂-Lösung ein pH-Wert von 6,5 erreicht wurde.
Die Färbung wurde, wie beschrieben, durchgeführt.
Es wurde wiederum eine gleichmäßige, intensive, glänzende Blondfärbung erhalten. Weglassen des Coenzyms Q 10 resultierte nicht nur in verschlechterten Haareigenschaften wie rauherer Griff, reduzierter Naß- und Trockenkämmbarkeit sowie geringerer Spannkraft, sondern vor allem auch in deutlich reduzierter Farbintensität, Gleichmäßigkeit der Färbung und weniger Glanz.

## Patentansprüche

1. Haarfärbemittel auf Basis eines Oxidationsfarbstoffvorprodukt-Systems, enthaltend mindestens eine Entwickler und mindestens eine Kupplersubstanz und 0,001 bis 10 Gew.-%, bezogen auf die Zusammensetzung, mindestens eines Ubichinons der Formel (I) 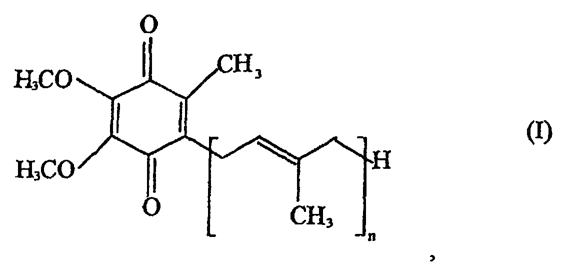 worin n eine Zahl von 1 bis 10 bedeutet

2. Mittel nach Anspruch 1, worin n 10 bedeutet.

3. Mittel nach Anspruch 1, worin n 6 bedeutet.

4. Mittel nach einem oder mehreren der Ansprüche 1 bis 3, enthaltend 0,01 bis 2,5 Gew.-% mindestens eines Ubichinons.

5. Verwendung eines Ubichinons der Formel (I) 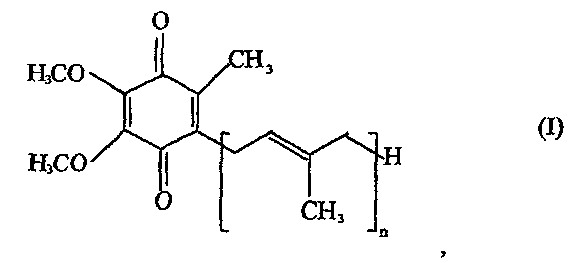 worin n eine Zahl von 1 bis 10 bedeutet,
in Haarfärbemitteln auf Basis von Oxidationsfarbstoff-Vorprodukten.

## Revendications

1. Composition de coloration pour les cheveux basé sur un système de produit précurseur de colorant d'oxydation, contenant au moins un révélateur et au moins un coupleur, et de 0,001 à 10 pour cent en poids, rapporté à la composition, d'au moins une ubiquinone de Formule (I) : 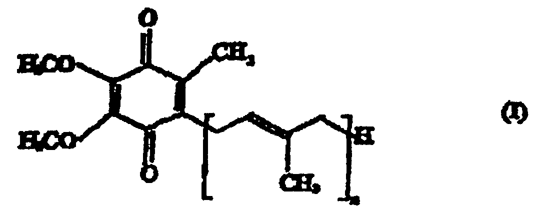 dans laquelle n représente un nombre de 1 à 10.

2. Produit selon la revendication 1, dans lequel n représente 10.

3. Produit selon la revendication 1, dans lequel n représente 6.

4. Produit selon l'une quelconque des revendications 1 à 3 contenant 0,01 à 2,5 pour cent en poids d'au moins une ubiquinone.

5. Utilisation, dans des compositions de coloration des cheveux, basée sur des produits précurseurs de colorant d'oxydation, d'une ubiquinone de formule (I) 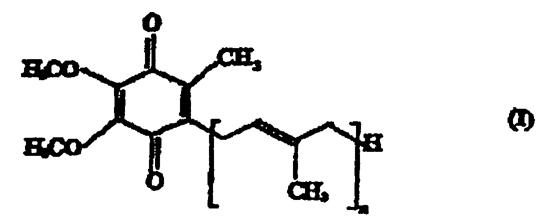 dans laquelle n représente un nombre de 1 à 10.

## Claims

1. Hair dyeing composition on the basis of an oxidation dyestuff precursor system, comprising at least one developing and at least one coupling substance and 0.001 % to 10 % by weight, in reference to the composition, of at least one ubiquinone of the formula (I) 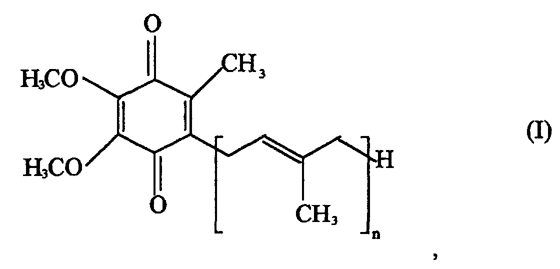 wherein n is a number from 1 to 10.

2. Composition according to claim 1, wherein n is 10.

3. Composition according to claim 1, wherein n is 6.

4. Composition according to one or more of claims 1 to 3, comprising 0.1 % to 2.5 % by weight of at least one ubiquinone.

5. Use of a ubiquinone of the formula (I) 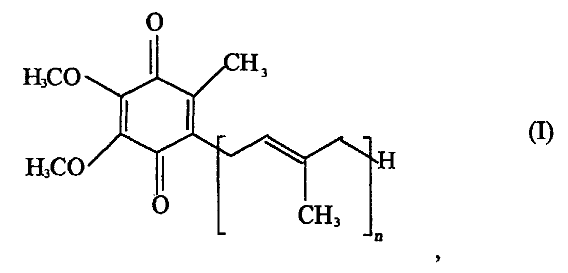 wherein n is a number from 1 to 10,
in hair dyeing compositions on the basis of oxidation dyestuff precursors.
